# EUROPEAN PATENT SPECIFICATION

(11) **EP 2 079 824 B1**
(45) Date of publication and mention of the grant of the patent: **18.05.2016**
(21) Application number: 07819930.4
(22) Date of filing: 10.08.2007
(51) Int. Cl.: A23L 33/115, A61K 31/25, C07C 67/03, C07C 69/58, C07C 69/587, C11C 1/02, C11C 3/00, C11C 3/10, C12P 7/62, C12P 7/64

(54) **MODIFIED ALKOXYGLYCEROLS**
MODIFIZIERTE ALKOXYGLYCEROLE
ALCOXYGLYCÉROLS MODIFIÉS

(30) Priority: 29.09.2006 ES 200602493
(43) Date of publication of application: 22.07.2009
(73) Proprietor: Universidad Autónoma de Madrid, 28049 Madrid (ES); Soluciones Extractivas Alimentarias, S.L., 50290 Epila Zaragoza (ES)
(72) Inventor: REGLERO RADA, Guillermo, 28049 Madrid (ES); VAZQUEZ FRUTOS, Luis, 28049 Madrid (ES); TORRES OLIVARES, Carlos, 28049 Madrid (ES); FORNARI REALE, Tiziana, 28049 Madrid (ES); SEÑORANS RODRIGUEZ, Francisco Javier, 28049 Madrid (ES); MORENO EGEA, Fernando, 28049 Madrid (ES)
(74) Representative: Carpintero Lopez, Francisco
(86) International application number: PCT/EP2007/058315
(87) International publication number: WO 2008/037538

(56) References cited:
- EP-A- 0 333 678
- AU-B2- 415 128
- TORRES C.F.; VAZQUEZ L.; SENORANS F.J.; REGLERO G.: "An efficient methodology for the preparation of alkoxyglycerols rich in conjugated linoleic acid and eicosapentaenoic acid" JOURNAL OF THE AMERICAN OIL CHEMISTS' SOCIETY, vol. 84, no. 5, 4 May 2007 (2007-05-04), pages 443-448, XP002480934
- HARALDSSON G.G.; THORARENSEN A.: "The generation of glyceryl ether lipids highly enriched with eicosapentaenoic acid and docosahexaenoic acid by lipase" TETRAHEDRON LETTERS, vol. 35, no. 41, 10 October 1994 (1994-10-10), pages 7681-7684, XP002480935
- HALLDORSSON A ET AL: "Lipase-catalysed kinetic resolution of 1-O-alkylglycerols by sequential transesterification" TETRAHEDRON: ASYMMETRY, ELSEVIER SCIENCE PUBLISHERS, AMSTERDAM, NL, vol. 15, no. 18, 20 September 2004 (2004-09-20), pages 2893-2899, XP004575069 ISSN: 0957-4166
- CATCHPOLE O.J. ; SIMOES P.; GREY J.B.; NOGUEIRO E.M.M.; CARMELO P.J.; DA PONTE M.N.: "Fractionation of lipids in a static mixer and packed column using supercritical carbon dioxide" INDUSTRIAL AND ENGINEERING CHEMISTRY RESEARCH, vol. 39, no. 12, December 2000 (2000-12), pages 4820-4827, XP002481175

## Description

### FIELD OF THE INVENTION

This invention relates to lipid compounds with biological activity and more specifically to lipid compounds derived from ether lipids (or alkoxyglycerols) with a beneficial activity for health, or which can be lipid carriers for the administration of bioactive ingredients. The invention further relates to the process for obtaining said modified alkoxyglycerols.

### STATE OF THE ART

Shark liver oil is a natural source of certain alkoxyglycerols and other types of lipids such as triacylglycerols, squalene and cholesterol. Natural alkoxyglycerols or 1-O-alkyl-sn-glycerols are valuable compounds which can be isolated from shark liver oil. Different applications of such compounds naturally present in fish oils, and particularly in shark liver oils, have been described, such as the use of these 1-O-alkyl-glycerols for preserving or improving sperm in pharmaceutical or veterinary uses (ES 2221057, W9800120). In this case, a 1-O-alkyl-glycerol compound chosen from the group comprising 16:0 1-alkyl-glycerol, 18:1 1-alkyl-glycerol and 16.1 1-alkylglycerol, which are ether lipids present in shark liver oil, is used.

Natural ether lipids (also known as alkoxyglycerols or alkyl glycerol ethers) have shown different beneficial effects for health. The oral administration of ether lipids reduces the negative effects of radiations received in radiation therapies. Prophylactic effects in certain tumors have also been observed. It has also been observed in experiments in both animals and humans that they are able to improve the immune response (Andreesen R., "Ether lipids in the therapy of cancer", Prog Biochem Pharmacol 22: 118-131, (1988), Palmblad, J, J Samuelsson, and J Brohult, "Interactions between alkylglycerols and human neutrophil granulocytes", Scand J Clin Lab Invest 50. 363-370 (1990)). EP 0 333 678 also discloses the use of glycerol ethers in the treatment of allergic diseases.

These ether lipids are found in bone marrow, breast milk, placenta and in shark liver oils in large amounts. In the human body, the ether lipids are mainly found in immune system cells and in breast milk. In some circumstances, the endogenous synthesis is reduced and in these cases, the administration of alkoxyglycerols is recommended.

To isolate natural alkoxyglycerols, it is necessary to eliminate other components of shark oil, such as squalene and fatty acids. Supercritical fluid technology, which has demonstrated very interesting properties, can be used to that end (Mukhopadhyay, M., Natural extracts using supercritical Carbon dioxide. CRC press. Boca Raton, Florida, 2000 and Catchpole O.J. et al. Ind. Eng. Chem. Res. 2000, 39. 4820-4827).

To produce fats and oils enriched with long chain polyunsaturated fatty acids and/or with conjugated linoleic acid, they can be enzymatically enriched.

Reactions catalyzed by esterification enzymes (Shimada, Y., A Sugihara, H Nakano, T. Kuramoto, T. Nagao. M. Gemba, and Y. Tominaga, Purification of Docosahexaenoic Acid by Selective Esterification of Fatty Acids from Tuna Oil with Rhizopus delemar Lipase, J Am. Oil Chem Soc 74: 97-101 (1997)), hydrolysis (McNeil, G. P, R.G Ackman, and B. Kristinsson, Lipase-catalyzed enrichment of long-chain polyunsaturated fatty acids, J Am Oil Chem Soc 73: 1403-1407(1996)) transesterification (Breivik, H., G.G Haraldsson, and B Kristinsson, Preparation of Highly Purified Concentrates of Eicosapentaenoic Acid and Docosahexaenoic Acid, J Am Oil Chem Soc. 74, 1425-1429, 1997)), and acidolysis (Torres, C.F., F. Munir, L.P Lessard, and Charles G. Hill Jr., Lipase-Mediated Acidolysis of Tristearin with CLA in a Packed-Bed Reactor: A Kinetic Study, J Am Oil Chem Soc. 79 655-661, (2002)) have been used to obtain selective enrichments of natural substances with conjugated linoleic acid (CLA) and with polyunsaturated fatty acids.

Haraldsson G.G. et al (Tetrahedron letters Vol. 35 No.41: 7681-7684) also discloses the generation of glycerol ethers highly enriched in EPA and DHA by lipase. In turn Halldorsson A. et al (Tetrahedron: Asymmetry 15, 2004: 2893-2899) discloses a lipase catalysed resolution of 1-O-alkylglycerols by sequential transesterification.

One of the fatty acids that has attracted most the attention in the recent years is conjugated linoleic acid (CLA), which is formed by a mixture of positional and geometric isomers of octadecadienoic acid with conjugated double bonds. Nutritional studies based on animal models have demonstrated a wide variety of beneficial effects derived from ingesting CLA in the diet. These effects include their anticarcinogenic, antiatherogenic, antiobesity properties and their immune system enhancing effect (Whigham, L.D., M.E. Cook, R.L. Atkinson, "Conjugated Linoleic Acid. Implications for Human Health", Pharmacol Res. 42: 503-510, (2000), Pariza, M.W., Y. Park, M.E. Cook, "The Biologically Active Isomers of Conjugated Linoleic Acid", Prog. Lipid Res. 40: 283-298, (2001)).

Since the middle of the 20^{th} century, research has been carried out aimed at learning the effects of the polyunsaturated fatty acids or PUFA (PolyUnsaturated Fatty Acids) in reducing serum cholesterol levels and in cardiovascular diseases. The most relevant works in this sense where those of Ahrens et al., 1954 (Ahrens E.H., D.H. Blankenhorn, T.T. Tastas (1954), "Effect on human serum lipids of substituting plant for animal fat in the diet", Proc. Soc. Exp. Biol. Med 86, 872) and Keys et al., 1957 (Keys A, J.T. Anderson, F. Grande (1957), "Serum cholesterol response to dietary fat", Lancet 1, 787) which provided clear evidence regarding the importance of PUFA in preventing cardiovascular diseases. Many studies have been conducted since then to that effect, most of which have confirmed the heart-healthy effects of omega-3. For example, in a clinical trial recently conducted by researchers at the Laboratory of Cardiovascular Nutrition of the Baker Medical Research Institute of Melbourne, the Department of Medicine of the Medical Defense College of Tokyo, the CSIRO of the Division of Health Sciences and Nutrition of Adelaide (Australia) and of Vitamin Research of F Hoffmann-La Roche (Switzerland) published in the American Journal of Clinical Nutrition (Am J Clin Nutr 76 (2002) 326-330) of the American Society for Clinical Nutrition, it has been demonstrated that omega-3 fatty acids, especially long-chain omega-3 fatty acids, i.e. DHA (docosahexaenoic acid) and EPA (eicosapentaenoic acid), have effects on the maintenance of arterial elasticity and, accordingly, on the maintenance of normal blood pressure levels and the reduction of cardiovascular risk. The study consisted of administering DHA or EPA or placebo to patients with hypercholesterolemia for seven weeks. The researchers then determined elasticity of the arteries of the participants through ultrasounds. Those who received ω-3 fatty acids showed a significant reduction in arterial sclerosis, whereas those who took placebo experienced no changes. Those who took EPA showed a 36% increase in arterial systemic resistance, a measure of elasticity of the main arteries, whereas those who took DHA showed a 27% increase.

Omega-3 fatty acids (EPA/DHA) improve the blood lipid profile because they increase elasticity, decrease LDL cholesterol, increase HDL cholesterol, reduce arterial triglycerides and are antithrombotic. Adeemia (López-Huertas-E; Baro, L.; Carrero, J J; Fonolla, J (2003) "n-3 fatty acids: health effects and opportunities to increase intake", Agro Food Industry hi tech 2003, 14(3). 18-21; Dewailly, E, Blanchet, C, Gingras S; Lemieux, S, Holub, B J (2002), "Cardiovascular disease risk factors and n-3 fatty acid status in the adult population of James Bay Cree", American Journal of Clinical Nutrition 2002; 76(1), 85-92).

In addition to the heart-healthy effects of omega-3, and as discussed in preceding paragraphs, these fatty acids have important effects on gene expressions and other biochemical body processes. Among other functions, omega-3 stands out due to its involvement in forming cell membranes. Most brain tissues are rich in omega-3 fatty acids. The current knowledge of these effects is included in an article by Donald B. Jump of the Department of Physiology, Biochemistry, and Molecular Biology of Michigan State University published in the Journal of Biological Chemistry of the American Society for Biochemistry and Molecular Biology (J Biol Chem 227 (2002) 8755-8758).

One way of assuring suitable intake of these fatty acids and of CLA is the production of lipids rich in these substances. Ether lipids can furthermore be suitable as lipid carriers to assure higher bioavailability and a better response of these substances. In addition, the combined administration of both, fatty acids and alkoxyglycerols, in a single molecule enhances the beneficial effect that both molecules have separately.

### OBJECT OF THE INVENTION

A first object of the invention is represented by modified alkoxyglycerols of general formula CH₂OR₁-CHOR₂-CH₂OR₃ where R₁, R₂ and R₃ independently represent a hydrogen; a saturated or unsaturated, linear or branched C₂-C₂₄ alkyl group, with or without double bonds, or a conjugated or unconjugated, saturated or unsaturated, linear or branched C₁-C₂₄ acyl group with or without double bonds where at least one of R₁, R₂ or R₃ represents an alkyl group and at least one of R₁, R₂ or R₃ represents a conjugated linoleyl group.

A second object is the process for obtaining said modified alkoxyglycerols comprising three steps: supercritical fractionation of the natural alkoxyglycerols, saponification and re-esterification thereof.

Finally, another object of the invention is also the use of the modified alkoxyglycerols of the invention to prevent cardiovascular diseases, as an immunostimulant or as an anticarcinogen. Modified alkoxyglycerols are also useful as carrier agents for other active ingredients and bioactive substances, making them suitable to form part of food compositions in functional foods or as an additive in pharmaceutical compositions.

### DESCRIPTION OF THE INVENTION

A first aspect of the present invention relates to modified alkoxyglycerols of general formula CH₂OR₁-CHOR₂-CH₂OR₃ where R₁, R₂ and R₃ independently represent a hydrogen; a saturated or unsaturated, linear or branched C₂-C₂₄ alkyl group, with or without double bonds; or a conjugated or unconjugated, saturated or unsaturated, linear or branched C₁-C₂₄ acyl group with or without double bonds where at least one of R₁, R₂ or R₃ represents an alkyl group and at least one of R₁, R₂ or R₃ represents a conjugated linoleyl group.

In a preferred embodiment of the invention the modified alkoxyglycerols R₁ is a saturated or unsaturated, linear or branched C₂-C₂₄ alkyl; R₂ is a hydroxyl group or an alkyl or acyl group, both being C₂-C₂₄ and R₃ is a hydroxyl group or an alkyl or acyl group, both being conjugated or unconjugated, saturated or unsaturated, linear or branched C₂-C₂₄ with or without double bonds where at least one of R₂ or R₃ represents an acyl group.

In a specific embodiment, the acyl group or groups come from saturated fatty acids with 1-10 carbons.

Furthermore, in a preferred embodiment the acyl groups have one or more unsaturations. It is also preferred that these unsaturations are conjugated as, for example, in the case of conjugated linoleic acid (CLA) which, as an acyl substituent in the general formula, would be in the form of conjugated linoleyl radical.

Modified alkoxyglycerols with long-chain polyunsaturated fatty acids (PUFA) of the omega-3 or omega-6 family, such as for example docosahexaenoic acid (DHA) or eicosapentaenoic acid (EPA), are also a preferred embodiment.

Finally, it is also preferred that the acyl group or groups in the modified alkoxyglycerols of the invention come from oleic acid, therefore representing an oleyl radical.

In addition, when R1, R2 or R3 represent an alkyl group it is preferred that they have an even number of carbons.

The modified alkoxyglycerols of the invention (referred to as such hereinafter) are useful for preventing cardiovascular diseases and are also useful as stimulants of the immune system and anticarcinogenic. This activity comes from the beneficial effect provided by the fatty acids, whereby modifying the alkoxyglycerols, and by the ether lipids themselves. The modified alkoxyglycerols of the invention can be used in both the food and pharmaceutical industry for preparing functional products and drugs.

In fact, the alkoxyglycerols of the invention can be used as carrier agents of other bioactive compounds in food or pharmaceutical compositions. For example, the modified alkoxyglycerols of the invention can act as carrier agents of fatty acids of the omega-3, omega-6, omega-9 (oleic) and/or CLA family. A synergistic activity can thus be reached between the alkoxyglycerols of the invention and fatty acids carried by them.

Another aspect of the invention is the process for obtaining the modified alkoxyglycerols of the invention. This process comprises:
a) countercurrent column supercritical fractionation of shark liver oil for obtaining a refined product rich in alkoxyglycerols and an extract rich in squalene,
b) saponification of the refined product rich in alkoxyglycerols of a) for producing an unsaponifiable fraction rich in non-esterified alkoxyglycerols, and
c) chemical or enzymatic re-esterification of the product of b) with aliphatic, saturated or unsaturated, conjugated or unconjugated, linear or branched chain C₂-C₂₄ monocarboxylic acids, wherein at least one of them is a conjugated linoleyl group.

As a step prior to supercritical fractionation, the shark liver oil can be subjected to another optional supercritical extraction step to extract squalene and other lipid products present in the oil, such as cholesterol or triacylglycerols, to obtain a refined product rich in even purer alkoxyglycerols.

### Supercritical fractionation and extraction process

Supercritical fractionation is carried out in a filled column to separate ether lipid components from edible oil samples containing them. Shark liver oil is particularly preferred due to its high content in alkoxyglycerols. The extraction is carried out with supercritical fluids, preferably carbon dioxide, on the alkoxyglycerols source (shark liver oil, or from a shark oil chemically or enzymatically modified or modified by saponification). The fractionation is carried out at an extraction pressure of between 90 and 320 bar, preferably 9-270 bar, and at a temperature of between 10°C and 90°C. A refined product rich in alkoxyglycerols (less than 4% of squalene) and an extract rich in squalene are obtained after fractionation.

### Saponification process

The refined product rich in alkoxyglycerols obtained after supercritical fractionation is still unsuitable for esterification because it still has a high percentage of triacylglycerols that can cause unwanted esterification and transesterification reactions in the presence of lipases. Therefore, saponification is carried out before this step to eliminate all the triacylglycerols and to obtain an unsaponifiable fraction highly rich in non-esterified alkoxyglycerols.

Saponification is carried out by means of treating the refined product rich in alkoxyglycerols with soda or potash or with an alkali in a basic medium. An unsaponifiable residue is obtained from this reaction, which product will be used in the transesterification step.

### Enzymatic or chemical transesterification process

Esterification of the alkoxyglycerols is preferably carried out enzymatically with a lipase in the presence of C₂-C₂₄ fatty acids or with esters thereof, wherein at least one of them is a conjugated linoleyl group, with which the alkoxyglycerols are to be esterified. The reaction catalyzed by the lipase takes place in two consecutive steps. An alkylglycerol monoester is formed during the first acylation and the alkylglycerol diester is formed during a second acylation. In other words, modified alkoxyglycerols, different from natural alkoxyglycerols, are thus obtained.

Eicosapentaenoic acid, docosahexaenoic acid, arachydonic acid, oleic acid, eicosaenoic acid, docosaenoic acid or mixtures thereof are preferably used as fatty acids in the context of the present invention for re-esterification.
EXAMPLE: Process for producing modified alkoxyglycerols with a high content of substituents of fatty conjugated linoleic acid (CLA) from shark liver oil

An extraction was carried out with supercritical fluids preferably of shark liver oil, or of a shark oil modified chemically or enzymatically or by saponification, followed by saponification of the refined product rich in alkoxyglycerols and a subsequent reaction of the unsaponifiable fraction with CLA or CLA ethyl ester.

The extraction with supercritical fluids eliminated more than 95% of the content of squalene from the shark oil, producing a refined product with a content in said hydrocarbon of less than 4% by weight.

This refined product was still unsuitable to produce modified alkoxyglycerols because it contained a high percentage of triacylglycerols, which could cause unwanted esterification and transesterification reactions in the presence of lipases. Therefore, a saponification reaction was carried out to effectively eliminate all the triacylglycerols and to obtain an unsaponifiable fraction highly rich in non-esterified alkoxyglycerols.

The transesterification was carried out between the ethyl ester of the conjugated linoleic acid (CLA) and the non-esterified alkoxyglycerols to produce alkoxyglycerols with conjugated linoleic acid derivatives. There was continuous nitrogen bubbling during the reaction to evaporate the ethanes caused by the reaction, which shifted the balance of the reaction towards the production of alkylglycerol diester with conjugated linoleic acid.

### Extraction with supercritical fluids

Supercritical fractionation is carried out with carbon dioxide in an installation with a filled column, in countercurrent conditions, to obtain a refined product with a 3% squalene content.

The extraction was carried out at a temperature of 60°C and at a pressure of 180 bar, with a solvent-feed ratio of 45.

### Saponification

5 grams of refined product were mixed with 16 mL of 3.7 N potassium hydroxide solution in ethanol (water 50:50 v:v with 0.15% of EDTA). The mixture was heated at 60°C for one hour at 300 rpm. The reaction was stopped by adding 4 mL of water and the unsaponifiable product was extracted from the aqueous phase with 3 portions of 20 mL of diethyl ether. The ethereal phase was then dried with sodium sulfate and evaporated under nitrogen to obtain the unsaponifiable residue.

### Transesterification reaction

The unsaponifiable fraction (500 mg) and the CLA ethyl ester (818 mg) or the free fatty acid (CLA) was added to a 30 mL flask with stirring. The lipase (10% by weight) was added, the flask was closed and placed in an orbital shaker (at 200 rpm) at 55°C. Samples were periodically obtained. The reaction products were analyzed by preparing methyl esters of the free fatty acids, to that end 1 mL of 0.2 mole methanolic HCL at 250 µL was added and was left to stand for 4h at 60°C.

After adding 200 µL of water, the mixture was extracted, dried and centrifuged to obtain the compounds which were analyzed by gas chromatography.

## Claims

1. Modified alkoxyglycerols of general formula CH₂OR₁-CHOR₂-CH₂OR₃ where R₁, R₂ and R₃ independently represent between a hydrogen group; a saturated or unsaturated, linear or branched C₂-C₂₄ alkyl group, with or without double bonds, or a conjugated or unconjugated, saturated or unsaturated, linear or branched C₁-C₂₄ acyl group with or without double bonds, **characterized in that** at least one of R₁, R₂ or R₃ represents an alkyl group and **in that** at least one of R₁, R₂ or R₃ represents a conjugated linoleyl group.

2. Modified alkoxyglycerols according to claim 1, where R₁ is a saturated or unsaturated, linear or branched C₂-C₂₄ alkyl; R₂ is a hydrogen, a C₂-C₂₄ alkyl group or a C₁-C₂₄ acyl group, and R₃ is a hydrogen, a C₂-C₂₄ alkyl group or a C₁-C₂₄ acyl group, both being conjugated or unconjugated, saturated or unsaturated, linear or branched with or without double bonds, **characterized in that** at least one of R₂ or R₃ represents an acyl group.

3. Modified alkoxyglycerols according to claim 1, **characterized in that** at least one of R₁, R₂ and R₃ represents a saturated acyl group of 1-10 carbons.

4. Modified alkoxyglycerols according to claim 1, **characterized in that** at least one of R₁, R₂ and R₃ is an acyl group with one or more unsaturations.

5. Modified alkoxyglycerols according to claim 1, **characterized in that** at least one of R₁, R₂ and R₃ represents an oleyl group.

6. Modified alkoxyglycerols according to claim 1, **characterized in that** at least one of R₁, R₂ and R₃ represents an acyl group corresponding to that of a long-chain polyunsaturated acid (PUFA) of the omega-3 or omega-6 family.

7. Modified alkoxyglycerols according to claim 1, **characterized in that** at least one of R₁, R₂ and R₃ represents an alkyl group with an even number of carbon atoms.

8. Modified alkoxyglycerols according to claim 1 for their use in preventing cardiovascular diseases.

9. Modified alkoxyglycerols according to claim 1 for their use as an immune system stimulant.

10. Modified alkoxyglycerols according to claim 1 for their use as an anticarcinogen.

11. A process for preparing modified alkoxyglycerols according to claim 1, **characterized in that** it comprises:
a) supercritical fractionation in countercurrent column of shark liver oil for obtaining a refined product rich in alkoxyglycerols and an extract rich in squalene,
b) saponification of the refined product rich in alkoxyglycerols of a) for producing an unsaponifiable fraction rich in non-esterified alkoxyglycerols, and
c) chemical or enzymatic re-esterification of the product of b) with conjugated or unconjugated, aliphatic, saturated or unsaturated, linear or branched chain C₂-C₂₄ monocarboxylic acids, wherein at least one of them is a conjugated linoleyl group.

12. A process according to claim 11, **characterized in that** the supercritical fluid used for fractionation is carbon dioxide.

13. A process according to claim 11, **characterized in that** the extraction pressure in the column is between 90 and 320, preferably 90 and 270 bar, and the extraction temperature is between 10°C and 90°C.

14. A process according to claim 11, **characterized in that** saponification is carried out with soda, with potash or in a basic medium with an alkali.

15. A process according to claim 11, **characterized in that** re-esterification is an enzymatic process and is carried out with lipase.

16. A process according to claim 11, **characterized in that** a C₂-C₂₄ monocarboxylic acid used in re-esterification is eicosapentaenoic acid, docosahexaenoic acid, arachidonic acid, oleic acid, eicosaenoic acid, docosaenoic acid or mixtures thereof.

17. The use of modified alkoxyglycerols according to any of claims 1-7 for the manufacture of food or pharmaceutical compositions to prevent cardiovascular diseases.

18. The use of modified alkoxyglycerols according to any of claims 1-7 for the manufacture of food or pharmaceutical compositions to stimulate the immune system.

19. The use of modified alkoxyglycerols according to any of claims 1-7 for the manufacture of food or pharmaceutical compositions with anticarcinogenic activity.

20. The use of modified alkoxyglycerols according to claims 1-7 as carrier agents of other or others bioactive compounds in food or pharmaceutical compositions.

21. The use according to claim 20 where bioactive compounds synergistically act with the alkoxyglycerols simultaneously acting as carrier agents.

22. The use according to claim 21 where the bioactive compounds are fatty acids of the omega-3 and/or CLA family.

## Patentansprüche

1. Modifizierte Alkoxyglycerine mit der allgemeinen Formel CH₂OR₁-CHOR₂-CH₂OR₃, wobei R₁, R₂ und R₃ unabhängig eine Wasserstoffgruppe, eine gesättigte oder ungesättigte, lineare oder verzweigte C₂-C₂₄-Alkylgruppe mit oder ohne Doppelbindungen oder eine konjugierte oder unkonjugierte, gesättigte oder ungesättigte, lineare oder verzweigte C₂-C₂₄-Acylgruppe mit oder ohne Doppelbindungen repräsentieren, **dadurch gekennzeichnet, dass** mindestens eins von R₁, R₂ oder R₃ eine Alkylgruppe repräsentiert und mindestens eins von R₁, R₂ oder R₃ eine konjugierte Linoleylgruppe repräsentiert.

2. Modifizierte Alkoxyglycerine nach Anspruch 1, wobei R₁ ein gesättigtes oder ungesättigtes, lineares oder verzweigtes C₂-C₂₄-Alkyl ist; R₂ ein Wasserstoff, eine C₂-C₂₄-Alkylgruppe oder eine C₁-C₂₄-Acylgruppe ist und R₃ ein Wasserstoff, eine C₂-C₂₄-Alkylgruppe oder eine C₁-C₂₄-Acylgruppe ist, die beide konjugiert oder unkonjugiert, gesättigt oder ungesättigt, linear oder verzweigt mit oder ohne Doppelbindungen sind, **dadurch gekennzeichnet, dass** mindestens eins von R₂ oder R₃ eine Acylgruppe repräsentiert.

3. Modifizierte Alkoxyglycerine nach Anspruch 1, **dadurch gekennzeichnet, dass** mindestens eins von R₁, R₂ und R₃ eine gesättigte Acylgruppe mit 1-10 Kohlenstoffen repräsentiert.

4. Modifizierte Alkoxyglycerine nach Anspruch 1, **dadurch gekennzeichnet, dass** mindestens eins von R₁, R₂ und R₃ eine Acylgruppe mit einer oder mehreren ungesättigten Bindungen repräsentiert.

5. Modifizierte Alkoxyglycerine nach Anspruch 1, **dadurch gekennzeichnet, dass** mindestens eins von R₁, R₂ und R₃ eine Oleylgruppe repräsentiert.

6. Modifizierte Alkoxyglycerine nach Anspruch 1, **dadurch gekennzeichnet, dass** mindestens eins von R₁, R₂ und R₃ eine Acylgruppe repräsentiert, die derjenigen einer langkettigen, mehrfach ungesättigten Säure (PUFA) der omega-3- oder omega-6-Familieentspricht.

7. Modifizierte Alkoxyglycerine nach Anspruch 1, **dadurch gekennzeichnet, dass** mindestens eins von R₁, R₂ und R₃ eine Acylgruppe mit einer geraden Anzahl an Kohlenstoffatomen repräsentiert.

8. Modifizierte Alkoxyglycerine nach Anspruch 1 zur Verwendung in der Verhütung von Herz-Kreislauf-Erkrankungen.

9. Modifizierte Alkoxyglycerine nach Anspruch 1 zur Verwendung als Immunsystemstimulans.

10. Modifizierte Alkoxyglycerine nach Anspruch 1 zur Verwendung als Antikarzinogen.

11. Verfahren zur Herstellung von modifizierten Alkoxyglycerinen nach Anspruch 1, **dadurch gekennzeichnet, dass** es umfasst:
**a)** überkritische Fraktionierung von Haifischleberöl in der Gegenstromsäule, um ein raffiniertes Produkt, das reich an Alkoxyglycerinen ist, und einen Extrakt, der reich an Squalen ist, zu erhalten,
**b)** Verseifen des raffinierten Produkts, das reich an Alkoxyglycerinen ist, von a) zur Produktion einer unverseifbaren Fraktion, die reich an unveresterten Alkoxyglycerinen ist, und
**c)** chemische oder enzymatische Umesterung des Produkts von b) mit konjugierten oder unkonjugierten aliphatischen, gesättigten oder ungesättigten, linearen oder verzweigtkettigen C₂-C₄-Monocarbonsäuren, wobei mindestens eine von ihnen eine konjugierte Linoleylgruppe ist.

12. Verfahren nach Anspruch 11, **dadurch gekennzeichnet, dass** das für die Fraktionierung verwendete überkritische Fluid Kohlendioxid ist.

13. Verfahren nach Anspruch 11, **dadurch gekennzeichnet, dass** der Extraktionsdruck in der Säule zwischen 90 und 320, vorzugsweise 90 und 270 bar liegt und die Extraktionstemperatur zwischen 10°C und 90°C liegt.

14. Verfahren nach Anspruch 11, **dadurch gekennzeichnet, dass** die Verseifung mit Natriumcarbonat, mit Kaliumcarbonat oder in einem basischen Medium mit einem Alkali durchgeführt wird.

15. Verfahren nach Anspruch 11, **dadurch gekennzeichnet, dass** die Umesterung ein enzymatisches Verfahren ist und mit Lipase durchgeführt wird.

16. Verfahren nach Anspruch 11, **dadurch gekennzeichnet, dass** die in der Umesterung verwendete C₂-C₂₄-Monocarbonsäure Eicosapentaensäure, Docosahexaensure, Arachidonsäure, Ölsäure, Eicosaensäure, Docosansäure oder Mischungen davon ist.

17. Verwendung von modifizierten Alkoxyglycerinen nach einem der Ansprüche 1 bis 7 zur Herstellung von Nahrungsmittel- oder pharmazeutischen Zusammensetzungen zur Verhütung von Herz-Kreislauf-Erkrankungen.

18. Verwendung von modifizierten Alkoxyglycerinen nach einem der Ansprüche 1 bis 7 zur Herstellung von Nahrungsmittel- oder pharmazeutischen Zusammensetzungen zur Stimulierung des Immunsystems.

19. Verwendung von modifizierten Alkoxyglycerinen nach einem der Ansprüche 1 bis 7 zur Herstellung von Nahrungsmittel- oder pharmazeutischen Zusammensetzungen mit antikarzinogener Wirkung.

20. Verwendung von modifizierten Alkoxyglycerinen nach den Ansprüchen 1-7 als Trägermittel von anderer bioaktiver Verbindung oder anderen bioaktiven Verbindungen in Nahrungsmittel- oder pharmazeutischen Zusammensetzungen.

21. Verwendung nach Anspruch 20, wobei bioaktive Verbindungen synergistisch mit den Alkoxyglycerinen wirken, die gleichzeitig als Trägermittel wirken.

22. Verwendung nach Anspruch 21, wobei die bioaktiven Verbindungen Fettsäuren der omega-3- und/oder CLA-Familie sind.

## Revendications

1. Alcoxyglycérols modifiés de formule générale CH₂OR₁-CHOR₂-CH₂OR₃ où R₁, R₂ et R₃ représentent indépendamment un groupe hydrogène ; un groupe alkyle en C₂ à C₂₄ linéaire ou ramifié, saturé ou insaturé, avec ou sans doubles liaisons, ou un groupe acyle en C₁ à C₂₄ linéaire ou ramifié, saturé ou insaturé, conjugué ou non conjugué, avec ou sans doubles liaisons, **caractérisés en ce qu'**au moins un de R₁, R₂ ou R₃ représente un groupe alkyle et **en ce qu'**au moins un de R₁, R₂ ou R₃ représente un groupe linoléyle conjugué.

2. Alcoxyglycérols modifiés selon la revendication 1, dans lesquels R₁ est un groupe alkyle en C₂ à C₂₄ linéaire ou ramifié, saturé ou insaturé ; R₂ est un atome d'hydrogène, un groupe alkyle en C₂ à C₂₄ ou un groupe acyle en C₁ à C₂₄ et R₃ est un atome d'hydrogène, un groupe alkyle en C₂ à C₂₄ ou un groupe acyle en C₁ à C₂₄, les deux étant linéaires ou ramifiés, saturés ou insaturés, conjugués ou non conjugués, avec ou sans doubles liaisons, **caractérisés en ce qu'**au moins un de R₂ ou R₃ représente un groupe acyle.

3. Alcoxyglycérols modifiés selon la revendication 1, **caractérisés en ce qu'**au moins un de R₁, R₂ et R₃ représente un groupe acyle saturé de 1 à 10 atomes de carbone.

4. Alcoxyglycérols modifiés selon la revendication 1, **caractérisés en ce qu'**au moins un de R₁, R₂ et R₃ représente un groupe acyle avec une ou plusieurs insaturations.

5. Alcoxyglycérols modifiés selon la revendication 1, **caractérisés en ce qu'**au moins un de R₁, R₂ et R₃ représente un groupe oléyle.

6. Alcoxyglycérols modifiés selon la revendication 1, **caractérisés en ce qu'**au moins un de R₁, R₂ et R₃ représente un groupe acyle correspondant celui d'un acide polyinsaturé à longue chaîne (PUFA) de la famille des oméga 3 ou des oméga 6.

7. Alcoxyglycérols modifiés selon la revendication 1, **caractérisés en ce qu'**au moins un de R₁, R₂ et R₃ représente un groupe alkyle ayant un nombre pair d'atomes de carbone.

8. Alcoxyglycérols modifiés selon la revendication 1, pour leur utilisation dans la prévention de troubles cardiovasculaires.

9. Alcoxyglycérols modifiés selon la revendication 1, pour leur utilisation en tant que stimulant du système immunitaire.

10. Alcoxyglycérols modifiés selon la revendication 1, pour leur utilisation en tant qu'anticancérigène.

11. Procédé de préparation d'alcoxyglycérols modifiés selon la revendication 1, **caractérisé en ce qu'**il comprend :
a) le fractionnement supercritique dans une colonne à contre-courant d'huile de foie de requin pour obtenir une produit raffiné riche en alcoxyglycérols et un extrait riche en squalène,
b) saponification du produit raffiné riche en alcoxyglycérols de a) pour la production d'une fraction non saponifiable riche en alcoxyglycérols non estérifiés, et
c) ré-estérification chimique ou enzymatique du produit de b) avec des acides monocarboxyliques en C₂ à C₂₄ à chaîne linéaire ou ramifiée, saturés ou insaturés, aliphatiques, conjugués ou non conjugués, au moins un d'entre eux étant un groupe linoléyle conjugué.

12. Procédé selon la revendication 11, **caractérisé en ce que** le fluide supercritique utilisé pour le fractionnement est le dioxyde de carbone.

13. Procédé selon la revendication 11, **caractérisé en ce que** la pression d'extraction dans la colonne est comprise entre 90 et 320, de préférence 90 et 270 bar, et la température d'extraction est comprise entre 10 °C et 90 °C.

14. Procédé selon la revendication 11, **caractérisé en ce que** la saponification est réalisée avec du carbonate de sodium, de la potasse ou dans un milieu basique avec un alcali.

15. Procédé selon la revendication 11, **caractérisé en ce que** la ré-estérification est un processus enzymatique et est réalisée avec une lipase.

16. Procédé selon la revendication 11, **caractérisé en ce qu'**un acide monocarboxylique en C₂ à C₂₄ utilisé dans la ré-estérification est l'acide éicosapentaénoïque, l'acide docosahexaénoïque, l'acide arachidonique, l'acide oléique, l'acide éicosaénoïque, l'acide docosaénoïque ou des mélanges de ceux-ci.

17. Utilisation d'alcoxyglycérols modifiés selon l'une quelconque des revendications 1 à 7, pour la fabrication de compositions alimentaires ou pharmaceutiques pour empêcher des maladies cardiovasculaires.

18. Utilisation d'alcoxyglycérols modifiés selon l'une quelconque des revendications 1 à 7, pour la fabrication de compositions alimentaires ou pharmaceutiques pour stimuler le système immunitaire.

19. Utilisation d'alcoxyglycérols modifiés selon l'une quelconque des revendications 1 à 7, pour la fabrication de compositions alimentaires ou pharmaceutiques ayant une activité anticancérigène.

20. Utilisation d'alcoxyglycérols modifiés selon l'une quelconque des revendications 1 à 7 en tant qu'excipients d'un ou de plusieurs autres composés bioactifs dans des compositions alimentaires ou pharmaceutiques.

21. Utilisation selon la revendication 20, dans laquelle les composés bioactifs agissent de manière synergique avec les alcoxyglycérols, agissant simultanément en tant qu'excipients.

22. Utilisation selon la revendication 21, dans laquelle les composés bioactifs sont des acides gras de la famille des oméga 3 et/ou des ALC.
